(19) Europäisches Patentamt
European Patent Office
Office européen des brevets

(11) Publication number: **0 332 047 A2**

(12) **EUROPEAN PATENT APPLICATION**

(21) Application number: **89103624.6**

(51) Int. Cl.[4]: **C07D 313/00**

(22) Date of filing: **02.03.89**

(30) Priority: **11.03.88 IT 1973688**

(43) Date of publication of application: **13.09.89 Bulletin 89/37**

(84) Designated Contracting States: **AT BE CH DE ES FR GB GR IT LI LU NL SE**

(71) Applicant: **C.R.C. Compagnia di Ricerca Chimica S.p.A.**
**Via Pesenalat, 6**
**I-33048 San Giovanni al Natisone (Udine)(IT)**

(72) Inventor: **Moimas, Flavio**
**Via d'Annunzio 81/A**
**Ronchi Dei Legionari (Gorizia)(IT)**
Inventor: **Clauti, Giuliano**
**Via S. Rocco 126/1**
**Udine(IT)**

(74) Representative: **Bianchetti, Giuseppe**
**Studio Consulenza Brevettuale Via Rossini, 8**
**I-20122 Milan(IT)**

# (54) A process for the separation of macrocyclic diastereomers.

(57) A process for the separation of the two diastereomers (3S,7R) and (3S,7S)-3,4,5,6,7,8,9,10,11,12-decahydro-7,14,16-trihydroxy-3-methyl-1H-2-benzoxacyclotetradecin-1-one (α- and β-zeranol), of formulae Ia and Ib:

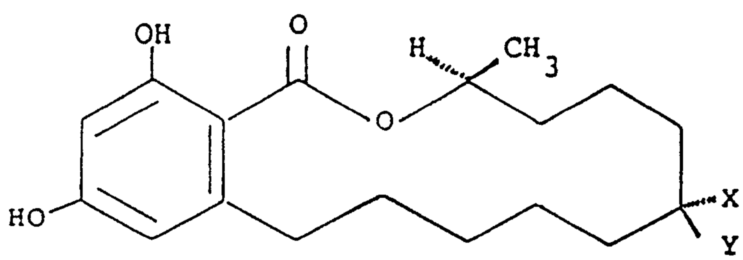

| | X | Y |
|---|---|---|
| Ia | HO | H |
| Ib | H | OH |

starting from the mixture thereof, which contains 50-60% diastereomer Ia, by means of crystallization from acetonitrile containing 0.2-2% water, under controlled cooling conditions, to obtain compound Ia ( α-zeranol) with a diastereomeric purity (titre) higher than 98.5%.

EP 0 332 047 A2

Xerox Copy Centre

## A PROCESS FOR THE SEPARATION OF MACROCYCLIC DIASTEREOMERS

The present invention relates to a process for the separation of the two macrocyclic diastereomers (3S,7R) and (3S,7S)-3,4,5,6,7,8,9,10,11,12-decahydro-7,14,16-trihydroxy-3-methyl-1H-2 benzoxacyclotetradecin-1-one, of formulae Ia and Ib:

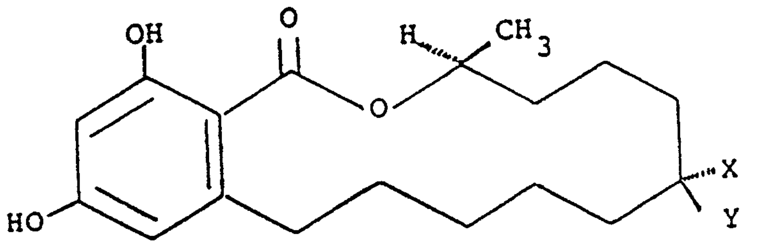

| | X | Y |
|---|---|---|
| Ia | HO | H |
| Ib | H | OH |

starting from the mixture thereof containing 50-60% of diastereomer Ia, by crystallization from hydrated acetonitrile, under controlled cooling conditions.

It is well known that of the two diastereomers Ia and Ib, only Ia (the α-zeranol form) has an high anabolic activity free from side-effects (U.S. 4.069.339; E. Maissoner and J.M. Vigneron - Editors - "Anabolics in Animal Production", Symposium OIE, Paris, 1983). Compound Ia with a titre higher than 98.5% is an extremely valuable product, specifically used in cattle-breeding (US Pat. 4.069.339).

The mixture of compounds Ia and Ib is obtained as the final product from a biotechnologic multi-step process, in which chemical transformations follow a fermentative part, which uses fungus Fusarium Graminearum on maize, to give unsaturated ketone II (Zearalenone).

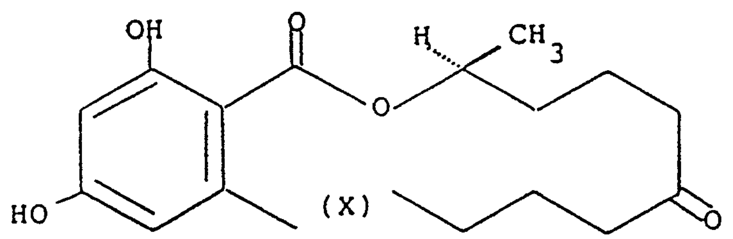

| | X |
|---|---|
| II | CH=CH |
| III | $CH_2$-$CH_2$ |

Said macrocyclic ketone is subjected to hydrogenation, thus reducing both the ketone carbonyl group and the CH = CH bond. It is well-known that reduction can be carried out either catalitycally (U.S. 3.239.345; Ger. Offen. 2.111.606, Ger. Offen. 2.253.467; Ger Offen. 2.328.605), or using complex hydrides in stoichiometric amounts (U.S. 3.704.294 (1972)), Italian Pat. 1.175.147, G. Shatzke et al., Helv. Chim. Acta 69 (1986) 734-748). Although the latter method gives a higher diastereo-selectivity, that is a higher content in the Ia form (α-), saving considerations undoubtedly tend to catalytic reduction, for example by means of nickel Raney, to the detriment of diastereoselectivity. In this case, the amount of Ia in the reduction product ranges from 50 to 60%. Separation of Ia from Ib and recycle of diastereomer Ib become therefore the crucial points for industrial carrying out of the whole process.

Separation of the two diastereomers Ia and Ib has been hitherto carried out by means of two completely different methods. The first one consists in acylating the Ia, Ib mixture with carboxylic acid-sulfuric acid, and recovering the mixture of the two 6′-O-acyl-diastereomers ( α- and β-forms), from which the α-6′-O-acyl isomer is separated by repeated crystallizations. Finally, the α-6′-O-acyl isomer is hydrolyzed and the obtained product is crystallized (US 3.687.982), to obtain compound Ia. The second method is disclosed in Italian Pat. Appl. No. 83329 A/85 in the Applicant's name, and essentially consists in the chromatographic separation, using HPLC, on industrial scale. This method, in spite of the better control of the plant and the savings in labour in comparison with the above cited one, requires a remarkable starting investment.

The process object of the present invention has substantial advantages from the plant and saving points of view in comparison with the up-to-now known methods.

In fact, it has been found that the Ia, Ib mixture from hydrogenation of Zearalenone, obtained by fermentation, or Zearalanone, having a Ia (α-Zeranol) titre from 50 to 60%, can be subjected to selective crystallization using an acetonitrile/water mixture as the crystallization solvent.

The water percentage in the solvent mixture can range from 0.1 to 10%, preferably from 0.2 to 2%. The solute/solvent ratio can be from 1:10 to 1:30; ratios from 1:20 to 1:22 being particularly preferred.

The process of the invention consists in heating to reflux the Ia, Ib mixture in hydrated acetonitrile and subsequently cooling, under controlled conditions (from about 5 to 10 °/hour), under continuous stirring, to a final temperature of 15 ° to 35 ° C.

Compound Ia crystallizes with a titre higher than 90% and in a 70-75% yield on the total amount of Ia. A second crystallization, carried out by conventional procedures, gives diastereomer Ia with a titre higher than 98.5%.

The second crystallization is preferably effected in a solvent-solute ratio from 25 to 35:1, more preferably from 28 to 30:1.

In the following table 1 the results of crystallization tests carried out with different acetonitrile/water ratios are reported. These data clearly show that maximum selectivity and yields correspond to a water content from 0.2 to 2%.

TABLE 1

| Titre and yield changes of Ia crystallized from acetonitrile/water (final temperature 30 ° C). | | | | | | |
|---|---|---|---|---|---|---|
| TEST | Ia:Ib mixture | $CH_3CN$-$H_2O$ | Dilution g/ml | Ia titre % HPLC | Weight yield | Ia yield |
| 1 | 58:42 | 10:5 | 1:10 | 92.0 | 24.3 | 38.5 |
| 2 | 58:42 | 10:1 | 1:10 | 93.0 | 25.5 | 40.9 |
| 3 | 58:42 | 9.9:0.1 | 1:20 | 92.0 | 39.0 | 61.9 |
| 4 | 58:42 | 10:0 | 1:22 | 83.5 | 47.2 | 67.9 |
| 5 | 54:46 | 10:1 | 1:10 | 91.0 | 21.6 | 36.4 |
| 6 | 54:46 | 9.95:0.01 | 1:22 | 92.8 | 42.3 | 72.7 |
| 7 | 54:46 | 9.98:0.02 | 1:20 | 91.6 | 40.8 | 69.2 |
| 8 | 54:46 | 9.98:0.02 | 1:22 | 92.0 | 43.1 | 73.4 |

Following table 2 shows how the final temperature and the solute/solvent ratio effect yield and diastereoselectivity. It is clear that the best results are obtained for ratios ranging from 1:20 to 1:22, whereas when the final temperature falls, yield increases to the detriment of selectivity.

TABLE 2

| Ia titre and yield changes as functions of dilution and final temperature (solvent:acetonitrile/water 99.8:0.2; starting mixture Ia:Ib = 54:46) | | | | | |
|---|---|---|---|---|---|
| TEST | DILUTION | Ia Titre (% HPLC) | Final temperature (° C) | Weight yield(%) | Ia yield (%) |
| 1 | 1:20 | 68.6 | 15 | 53.0 | 67.3 |
| 2 | 1:20 | 73.0 | 20 | 52.6 | 71.1 |
| 3 | 1:20 | 89.0 | 27 | 43.0 | 70.9 |
| 4 | 1:20 | 90.8 | 30 | 39.5 | 65.8 |
| 5 | 1:22 | 92.0 | 25 | 40.3 | 62.7 |
| 6 | 1:22 | 94.0 | 30 | 42.4 | 73.8 |
| 7 | 1:25 | 86.0 | 20 | 39.7 | 63.2 |
| 8 | 1:25 | 91.7 | 25 | 35.0 | 59.4 |
| 9 | 1:25 | 93.7 | 30 | 32.8 | 56.9 |

The product recovered from mother liquors, enriched in diastereomer Ib ($\beta$-Zeranol), can then in part be directly recycled to the crystallization step and in part be subjected to oxidation to Zearalanone, unlike the method desclosed in U.S. Pat. 3.687.982, which envisages a separated hydrolysis of the $\beta$-6′-O-acyl derivative to Ib and subsequent reoxidation.

According to another particularly preferred aspect of the invention, oxidation of the product recovered from mother liquours to Zearalanone is carried out using nickel Raney in t-butyl acetate up to a 75-80% conversion. In fact, it has been found that said oxidation is diastereoselective (i.e. isomer Ib is preferably oxidated): if oxidation is not completed, a mixture of Zearalanone and of Ia, Ib mixture enriched in Ia is therefore obtained.

The subsequent reduction of said mixture leads to a further enrichment in isomer Ia until a percentage even higher than 60%.

The following examples illustrate the conditions and experimental techniques used in the present invention, not limiting anyway the scope and spirit thereof.

## EXAMPLE 1

Separation of Ia ( α-Zeranol) from the Ia, Ib mixture

4.5 g of Ia, Ib mixture (50:50) were placed in acetonitrile containing about 1% water (100 ml) and active charcoal (0.1 g) was added. The mixture was refluxed for 15 min., then the warm solution was filtered on Celite; the filtrate was heated to obtain a clear solution, that is to about 80° C. The solution was cooled under stirring, adjusting the fall in temperature to about 6-7° C/hour, thereby the solution temperature after about 8 hours being 30° C. The precipitate was filtered, washed with 10 ml of cold acetonitrile, finally dried. 1.8 g (40%) of Ia ( α-zeranol) was obtained, with 91% purity (determined by HPLC, using a Nucleosil C18 column, 4.6 mm x 200 mm; eluent: acetonitrile/methanol/water 41:14:45, adjusted to pH 3.5 with phosphoric acid; retention times: Ia 6.28 min., Ib 5.06 min.: developer UV-VIS,λ = 265 nm).

The whole amount of Ia (1.8 g) was dissolved in 55 ml of acetonitrile/water (99.5:0.5); heating to reflux, then filtered on Celite and the filtrate was heated to 80° C. The clear solution was cooled adjusting the fall in temperature to about 6-7° C/hour; during about 8 hours the solution was cooled to 30° C, under stirring. The solid was filtered and washed with 5 ml of cold acetonitrile; after drying 1.26 g of Ia was obtained, with a 98,5% diastereomeric purity.

Titre was determined by analytio HPLC, as described above; m.p. 180-182° C; $[\alpha]_D^{20}$ = +46.2 (c = 1.0; methanol).

## EXAMPLE 2

Separation of Ia (α -Zeranol) from the Ia, Ib mixture

3.59 kg of a Ia, Ib mixture (55:45), acetonitrile with 0.5% water (80 1) and active charcoal (0.05 kg) were placed into a 150 1 crystallizer. The mixture was refluxed for 30 min., warm filtered and the filtrate was refluxed again. The obtained clear solution was cooled adjusting the fall in temperature to about 8-10° C/hour, under strong mechanic stirring, so as to obtain a controlled crystallization. Crystal withdrawals were carried out at 42° C, 36° C and 30° C, on which crystals HPLC analysis was effected as described in Example 1: content in Ia proved to be extremely constant (91.9%, 92.3% and 91.8%, respectively, at the above cited temperatures). Stirring was continued for 12 hours more at 30° C (besides the night); then the product was filtered and dried to obtain 1.40 kg (38.99%) of Ia with a 93.5% titre. This product was dissolved in 42 1 of acetonitrile with 1% of water and crystallized following the same procedure as above. 1.01 kg of final product was obtained, with a 98.6% titre (HPLC), m.p. = 181-182° C, $[\alpha]_D^{20}$ = 46.2 (c = 1.0; methanol).

## EXAMPLE 3

Recycle of the Ia, Ib mixture (Ia titre about 30%) via oxidation

Mother liquors from the first crystallization described in Example 2 (80 1) were concentrated to small volume (about 10 1). The suspension was filtered to give 1.92 kg of a Ia, Ib mixture with a Ia titre of 29.2% (determined via HPLC, as in Example 1). This product was oxidized with nickel Raney (2.5 kg) in n-butyl acetate (30 1). After refluxing for 6 hours, HPLC shows a content in III (Zearalanone) of about 80%, in Ia of about 12-14% and in Ib of 6-8%. (For quantitative determination, a Bio-sil ODS-5S column, 4 mm x 150 mm was used; eluent as in Example 1; retention times: III = 5.04 min., Ia = 3.51 min., Ib - 2.63 min.). The catalyst was filtered off and washed with 10 1 of n-butyl acetate; then the solution was evaporated to 5 1, obtaining 1.6 kg of a mixture of Ia, Ib, III, as above described. Said mixture was hydrogenated together with II (Zearalenone) from Fusarium fermentation, to give a Ia, Ib mixture with Ia titre from 60 to 65%.

## EXAMPLE 4

Recycle of the Ia, Ib mixture (Ia titre of about 30%) via crystallization

Mother liquors from the first crystallization described in Example 2 (80 1) were concentrated to small volume, then the precipitate consisting of the Ia, Ib mixture (1.92 kg) with a Ia titre of 29.2%, was filtered.

This mixture wad admixed with a Ia, Ib mixture (4.36 kg, Ia titre 71%), obtained from mother liquors from the second crystallization. The final mixture (6.28 kg) contained Ia in a 58.2% percentage. This mixture was crystallized with the Ia, Ib mixture from catalytic reduction of Zearalenone, as described in Example 2.

## Claims

1. A process for the separation of the diastereomers (3S,7R) and (3S,7S)3,4,5,6,7,8,9,10,11,12-decahydro-7,14,16-trihydroxy-3-methyl-1H-2-benzoxacyclotetradecin-1-one, of formulae Ia and Ib:

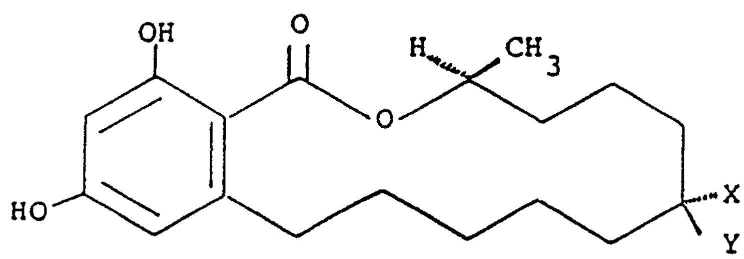

| | X | Y |
|---|---|---|
| Ia | HO | H |
| Ib | H | OH |

starting from the mixture thereof containing 50-60% diastereomer Ia, which process consists in subjecting said mixture to crystallization from acetonitrile containing 0.1 to 10% water.

2. A process as claimed in claim 1, in which 0.2 to 2% water is present.

3. A process as claimed in claim 1 or 2, in which the solvent:solute ratio is 15-30:1, preferably 20-22:1.

4. A process as claimed in any one of claims 1-3, in which the solution of Ia, Ib mixture in acetonitrile/water is cooled at a 5-10°C/hour rate, up to a final temperature of 25-35°C, particularly to 30°C.

5. A process as claimed in any one of claims 1 to 4, in which the product obtained from the first crystallization is subjected to a second crystallization from acetoni trile/water in a solvent:solute ratio of 25-35:1, preferably of 28-30:1, to give product Ia with a diastereomeric purity higher than 98.5%.

6. A process as claimed in any one of claims 1 to 5, in which the Ia, Ib mixture (about 30:70), obtained from mother liquors after the first crystallization, and the Ia, Ib mixture obtained from the second crystallization (Ia to Ib ratio about 70:30) are combined to give a final mixture with a Ia titre of 50-60%, which mixture can be combined with the starting Ia, Ib mixture.

7. A process as claimed in any one of claims 1 to 5, in which part of the Ia, Ib mixture (ratio about 30:70) obtined from mother liquors after the first crystallization, is oxidized with nickel Raney in n-butyl acetate under reflux for 6-8 hours, until conversion not higher than 75-80%, and the obtained mixture is finally recycled by hydrogenation together with Zearalenone (II).